# EUROPÄISCHE PATENTSCHRIFT

(11) **EP 2 094 116 B1**
(45) Veröffentlichungstag und Bekanntmachung des Hinweises auf die Patenterteilung: **19.05.2010**
(21) Anmeldenummer: 07819368.7
(22) Anmeldetag: 26.10.2007
(51) Int. Cl.: A23L 1/305, A61K 31/198

(54) **FESTE ODER WÄSSRIGE ALKALISCHE ZUBEREITUNG UMFASSEND EINE KREATIN-KOMPONENTE, VERFAHREN ZU DEREN HERSTELLUNG UND IHRE VERWENDUNG**
SOLID OR AQUEOUS ALKALINE PREPARATION COMPRISING A CREATINE COMPONENT, PROCESS FOR THE PRODUCTION THEREOF AND THE USE THEREOF
PRÉPARATION ALCALINE SOLIDE OU AQUEUSE COMPRENANT UN COMPOSANT DE CRÉATINE, LEUR PROCÉDÉ DE FABRICATION ET LEUR UTILISATION

(30) Priorität: 28.10.2006 DE 102006050931
(43) Veröffentlichungstag der Anmeldung: 02.09.2009
(73) Patentinhaber: AlzChem Trostberg GmbH, 83308 Trostberg (DE)
(72) Erfinder: GASTNER, Thomas, 84518 Garching an der Alz (DE)
(74) Vertreter: Böhm, Brigitte
(86) Internationale Anmeldenummer: PCT/EP2007/009324
(87) Internationale Veröffentlichungsnummer: WO 2008/052712

(56) Entgegenhaltungen:
- WO-A-02/069740
- WO-A-2005/120246
- DE-A1- 19 830 768
- US-A- 5 908 864
- US-B1- 6 399 661

## Beschreibung

Gegenstand der vorliegenden Erfindung ist eine feste oder wässrige alkalische Zubereitung umfassend eine Kreatin-Komponente, Verfahren zu ihrer Herstellung sowie deren Verwendung als Nahrungsergänzungsmittel, Stärkungsmittel, medizinische Präparate sowie Futtermittel.

Im Jahr 1832 wurde von dem französischen Chemiker Chevreul eine neue Verbindung aus Fleischbrühe isoliert. Cheuvreul nannte diese Verbindung Kreatin und orientiert sich hierbei an dem griechischen Wort für Fleisch ("Kreas"). Diese Arbeiten wurden 15 Jahre später von Justus von Liebig wieder aufgenommen, wobei er zeigen konnte, dass Kreatin ein natürlicher Bestandteil des Muskelsaftes von Wirbeltieren ist. Der später von Liebig hergestellte Fleischextrakt war das erste kommerziell erhältlich Nahrungsmittel, welches Kreatin in konzentrierter Form (ca. 10 Gew.-%) enthielt. Zu der Zeit gab es in Südamerika einen großen Überschuss an Rindfleisch, da dieses aufgrund fehlender Kühlmöglichkeiten nicht über weitere Strecken transportiert werden konnte. Die Tiere wurden damals vor allem wegen der Gewinnung ihrer Häute, Hörner und Knochen gehalten. Die Erfindung des Fleischextrakts war ein großer Markterfolg, da hierdurch auch das Fleisch der Tiere sinnvoll genutzt werden konnte. Die Fleischbrühe gewann später auch durch die Kriege in Europa stark an Bedeutung und wurde als nährstoffreiche Kraftnahrung für Soldaten verwendet. Der von Liebig entwickelte Fleischextrakt ist auch noch heute zur geschmacklichen Anreicherung von Suppen und Soßen beliebt.

Seit Ende der 70iger Jahre des vergangenen Jahrhunderts wird die erwogene Wirkung von Kreatin systematisch untersucht. Bis heute wurden über 300 Studien im Sportbereich durchgeführt, wobei etwa 80 % dieser Studien signifikant positive Effekte des Kreatins auf die Muskelmasse, die Muskelkraft, die fettfreie Körpermasse und die Leistung bei maximaler, kurzzeitiger Muskelanstrengung in verschiedenen Sportarten zeigte. Kreatin-Monohydrat ist heute das wichtigste Nahrungsergänzungsmittel im Sportbereich.

Erst vor kurzer Zeit sind weitere interessante Eigenschaften des Kreatins bekannt geworden. So wurden in zwei Studien signifikant positive Wirkungen einer oralen Kreatin-Supplementation auf die Gehimleistung und die Konzentrationsfähigkeit nachgewiesen (Rae, Caroline et al.: Oral creatine monohydrate supplementation improves brain performance: a double-blind, placebo-controlled, cross-over trial. Proceedings of the Royal Society of London, Series B: Biological Sciences (2003), 270(1529), 2147-2150; Watanabe, Airi et al.: Effects of creatine on mental fatigue and cerebral hemoglobin oxygenation. Neuroscience Research (Oxford, United Kingdom) (2002), 42(4), 279-285).

Weiterhin konnte gezeigt werden, dass Kreatin antioxidative und neuroprotektive Eigenschaften besitzt und somit auch zur Vorbeugung von Schädigungen der Zellen durch Umwelteinflüsse eingesetzt werden kann (Sestili, Piero et al.: Creatine supplementation affords cytoprotection in oxidatively injured cultured mammalian cells via direct antioxidant activity. Free Radical Biology & Medicine (2006), 40(5), 837-849; P. Klivenyi et al.: Neuroprotective effects of creatine in a transgenic animal model of amyotrophic lateral sclerosis. Nature Medicine 5, 347-350 (1999)). Kreatin wird deshalb in Zukunft auch im Anti-Aging Bereich stark an Bedeutung gewinnen.

Die positiven Effekte von Kreatin werden derzeit auch im medizinischen Bereich intensiv untersucht, wobei sich Kreatin bei der Behandlung von Parkinson und der Amyotrophen Lateralen Sklerose (ALS) in der klinischen Phase 3 und bei Corea-Huntington in Phase 2 befindet (EP 804 183 B1). Auch von einem erfolgreichen Einsatz von Kreatin als Therapeutikum gegen Asthma wurde bereits berichtet (EP 911 026 B1). Beim Aufbau von Knochen zeigte Kreatin sowohl in vitro als auch in vivo positive Effekte. Der Einsatz zur Stärkung der Knochen und zur Behandlung und Vorbeugung von degenerativen Knochen- und Knorpel-Erkrankungen wie etwa Osteoporose wurde untersucht und lieferte sehr positive Ergebnisse (EP 1 100 488 B1; Gerber, I et al.: Stimulatory effects of creatine on metabolic activity, differentiation and mineralization of primary osteoblast-like cells in monolayer and micromass cell cultures. European Cells and Materials (2005), 10, 8-22; Chilibeck, P. D. et al.: Creatine monohydrate and resistance training increase bone mineral content and density in older men. Journal of Nutrition, Health & Aging (2005), 9(5), 352-355).

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig gesehen führt das Kreatin aber indirekt durch vermehrte Proteinsynthese oder einen verminderten Proteinkatabolismus in den Myofibrillen zu einer Erhöhung der Muskelmasse (Int J Sports Med 21 (2000), 139-145). Als Ergebnis erhält man somit eine erhöhte fettfreie Körpermasse.

Neben dem Kreatin selbst, also dem Kreatin-Monohydrat, haben sich zwischenzeitlich aber auch zahlreiche Kreatin-Salze, wie das Kreatinascorbat, -citrat, -pyruvat, -phosphat und andere, ebenfalls als geeignete Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Der Metabolismus und die Wirkungsweise von Kreatin sind sehr gut untersucht. Die Biosynthese geht von Glycin und L-Arginin aus. Bei Säugetieren wird vor allem in den Nieren, aber auch in der Leber und der Bauchspeicheldrüse, die Guanidino-Gruppe des L-Arginins durch das Enzym Aminotransferase gespalten und eine N-C-N-Gruppe auf das Glycin übertragen. Das L-Arginin wird hierbei in L-Omithin umgewandelt. Die so gebildete Guanidinoessigsäure wird im nächsten Schritt, der bei Vertebraten vor allem in der Leber erfolgt, mit Hilfe des Enzyms Transmethylase in Kreatin umgewandelt. Hierbei dient das S-Adenosylmethionin als Methylgruppen-Donor. Das Kreatin diffundiert anschließend in den Blutkreislauf und wird so zu den Zielorganen transportiert. Der Transport durch die Zellmembran in die Zellen geschieht hierbei durch einen spezifischen NaCl-abhängigen Kreatin-Transporter (Speer O, Neukomm LJ, Murphy RM, Zanolla E, Schlattner U, Henry H, Snow RJ, Wallimann T. Creatine transporters: a reappraisal. Mol Cell Biochem. 2004 Jan-Feb;256-257(1-2):407-24).

Kreatin spielt im Energiestoffwechsel der Zelle eine wichtige Rolle, wobei es als energiereiches Phosphokreatin neben dem Adenosintriphosphat (ATP) eine wesentliche Energiereserve des Muskels darstellt. Im Ruhezustand des Muskels kann ATP eine Phosphat-Gruppe auf Kreatin übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion, durch das Enzym Kreatinkinase eine Phosphatgruppe auf Adenosindiphosphat übertragen und somit ATP zurückbilden. Dies wird auch als Lohmann-Reaktion bezeichnet.

Weiterhin besitzt Kreatin eine wichtige Funktion bei der Übertragung von Energie in der Zelle. Das so genannte Kreatin-Shuttle-System transportiert Energie von den Mitochondrien an die Stellen in der Zelle, an denen die Energie benötigt wird.

Bei starker und über längere Zeit anhaltender Muskelarbeit sind die natürlichen, im Körper vorhandenen Kreatin-Vorräte rasch erschöpft. Aus diesem Grund haben sich insbesondere bei Leistungssportlem gezielte Kreatin-Gaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper über die Nieren ausgeschieden wird. Weiterhin wandelt sich Kreatin mit einer konstanten Rate in das zyklische Abfallprodukt Kreatinin um, welches ebenfalls über die Nieren ausgeschieden wird und somit einen zweiten metabolischen Abbauweg darstellt.

Die Aufnahme von Kreatin in die Muskulatur wird von einem NaCl-abhängigen Kreatin-Transporter gesteuert und kann durch die gleichzeitige Aufnahme von Kohlenhydraten und Proteinen positiv beeinflusst werden. Dabei zeigte sich, dass die Kombination von Kreatin und Kohlenhydraten im Vergleich zur alleinigen Einnahme von Kreatin zu einem um 60 % erhöhten Anstieg des Kreatin-Gehalts im Muskel führen kann (Green AL, Hultman E, Macdonald IA, Sewell DA, Greenhaff PL. Carbohydrate ingestion augments skeletal muscle creatine accumulation during creatine supplementation in humans. Am J Physiol. 1996 Nov;271 (5 Pt 1):E821-6). Es konnte gezeigt werden, dass die Ausschüttung von Insulin bei der Aufnahme von Kreatin in die Muskelzellen eine wichtige Rolle spielt. Zwischen der Erhöhung der Kreatin-Konzentration in der Muskulatur und der ausgeschütteten Menge an Insulin besteht ein linearer Zusammenhang (Steenge GR, Simpson EJ, Greenhaff PL. Protein- and carbohydrate-induced augmentation of whole body creatine retention in humans. J Appl Physiol. 2000 Sep;89(3):1165-71).

Neben seinen unbestrittenen positiven physiologischen Eigenschaften besitzt Kreatin aber auch den Nachteil, dass es in den entsprechenden wässrigen Lösungen keine ausgeprägte Stabilität besitzt. Kreatin zyklisiert hierbei durch die Abspaltung von Wasser zum Kreatinin. Die Zyklisierungsgeschwindigkeit ist vom pH-Wert der Lösung und der Temperatur abhängig, wobei die Konzentration keine Rolle spielt. Besonders im neutralen und sauren pH-Bereich verläuft die Umwandlung in Kreatinin sehr schnell. Aufgrund des schnellen Abbaus von Kreatin in diesem Milieu ist der Einsatz in wässrigen oder feuchten Formulierungen für die menschliche und tierische Ernährung praktisch ausgeschlossen. Bereits der pH-Wert des Magens von 1 bis 2 kann je nach Verweilzeit zu einem deutlichen Abbau des Kreatins zu Kreatinin führen. (Greenhaff, P.L.: Factors Modifying Creatine Accumulation in Human Skeletal Muscle. In: Creatine. From Basic Science to Clinical Application. Medical Science Symposia Series Volume 14, 2000, 75-82).

Die Stabilität von Kreatin in Abhängigkeit vom pH-Wert wurde bereits 1928 eingehend untersucht und die höhere Stabilität im alkalischen Bereich ist bereits seit dieser Zeit bekannt (Cannan, Robert Keith; Shore, Agnes. Creatine-creatinine equilibrium. The apparent dissociation constants of creatine and creatinine. Biochemical Journal (1928), 22, 920-9). Die Verwendung eines alkalischen Kreatins, für Zubereitungen die der Ernährung dienen, wurde aber erst viel später beschrieben.

So beansprucht EP 669 083 A2 ein alkalisches Kreatin-Getränk und dessen Herstellung, welches sich durch die Stabilität des Kreatins beim Konservierungsprozess auszeichnet. Der Schutzbereich erstreckt sich hierbei auch auf ein Verfahren, bei dem 1.) Wasser mit einem basischen pH vorgelegt und erwärmt wird, 2.) 1-3 g Kreatin pro 100 ml unter Rühren gelöst werden und 3.) Additive zur Erhöhung des Nährstoffgehalts und Verbesserung des Geschmacks zugesetzt werden. Eine spezielle Base zur Einstellung des pH-Wertes wird in dieser Anmeldung nicht beschrieben.

US 6,399,661 beansprucht eine Kreatin-Zubereitung die dem Ernährungszweck dienen soll. Die beanspruchte Herstellung verläuft über ein dreistufiges Verfahren, bei dem 1.) ein alkalisches Pulver mit pulverförmigem Kreatin gemischt wird, um eine Mischung mit pH 7 bis 14 zu erhalten; 2.) ein pulverförmiges Additiv zugegeben wird, um Süße und Geschmack der Mischung zu verbessern und 3.) ein weiteres alkalisches Pulver zugegeben wird, um den pH-Wert der Mischung auf Werte zwischen 7 und 14 einzustellen. Als Base wird vorzugsweise Natriumcarbonat und/oder Magnesium-glycerolphoshat verwendet. Weiterhin kann die alkalische Komponente aus der Gruppe der Hydroxide, Carbonate, Bicarbonate, Chloride, Baumlatex oder Phosphate gewählt werden.

EP 1 520 580 A1 beansprucht eine Methode zur Steigerung der Ausdauer bei Säugetieren und beim Menschen, durch die Verwendung einer Kreatin-Zubereitung, welche einen pH zwischen 7 und 14 besitzt. Die verwendeten Zubereitungen entsprechen den in US 6,399,661 vorbeschriebenen Mischungen.

In US-A-5 908 864 wird ein gelförmiges kreatinhaltiges Nahrungsergänzungsmittel beschrieben. Zur Einstellung des pH-Wertes auf 6.5-8 wird Kaliumphosphat eingesetzt.

WO 02/069740 beschreibt eine Zusammensetzung für den menschlichen Verzehr bestehend aus Kreatin und Kreatinin, welches zur Stabilisierung des Kreatins in wässriger Lösung eingesetzt wird. Gemäß Beispiel 5 wird eine Lösung offenbart, die Kreatin-Monohydrat und ein Kaliumphosphalpuffersystem enthält, das einen pH-Wert von 8,0 und 8,25 einstellt.

Nachteilig bei den Zubereitungen gemäß dem Stand der Technik ist die Tatsache, dass schon geringe Mengen an Säuren ausreichen, um diese Mischungen zu neutralisieren oder auf einen sauren pH-Wert einzustellen. In der Praxis werden maximale Dosierungen von einigen Gramm derartiger Kreatin-Zubereitungen gewählt. Diese sind nach dem Auflösen in Wasser zunächst stabil, nach der oralen Aufnahme wird eine solche Dosis aufgrund der geringen Menge an enthaltener Base sehr schnell durch die Magensäure auf einen sauren pH-Wert eingestellt und das Kreatin wird somit instabil.

Die nach dem Stand der Technik bekannten basischen Kreatin-Zubereitungen werden somit dem Körper nicht in maximal möglicher Menge zur Verfügung gestellt, da weiterhin im sauren Milieu des Magens ein Teil des Kreatins in Kreatinin umgewandelt wird.

Aus den bzgl. der Stabilität des Kreatins geschilderten Nachteilen des Standes der Technik hat sich für die vorliegende Erfindung die Aufgabe gestellt, Zubereitungen zu entwickeln, welche das Kreatin besser vor dem Abbau zu Kreatinin im Magen schützen. Entscheidend ist hierbei eine optimale Versorgung der Körperzellen mit Kreatin, ohne dass hierbei Kreatinin gebildet wird, welches für den Körper nutzlos ist und somit über die Nieren aus dem Körper ausgeschieden werden muss.

Gelöst wurde diese Aufgabe durch die Bereitstellung einer Zubereitung gemäß Anspruch 1. In einer bevorzugten Ausführungsform der Erfindung ist die Zubereitung eine alkalische Zubereitung, die besonders bevorzugt fest oder wässrig ist.

Es konnte gezeigt werden, dass mit diesen Formulierungen die Aufgabenstellung voll erfüllt werden konnte, nämlich das Kreatin durch das Puffersystem im Magen vor einer Umwandlung in Kreatinin besser zu schützen. Überraschend hat sich herausgestellt, dass die neuen Formulierungen eine deutlich höhere Bioverfügbarkeit aufweisen und somit besser in die Zellen aufgenommen werden. Außerdem besitzt die erfindungsgemäße Zubereitung sehr gute organoleptische Eigenschaften, was ebenfalls nicht vorhersehbar war.

Die Zubereitung gemäß der vorliegenden Erfindung besteht aus einer Kreatin-Komponente und einem Puffersystem, wobei das Puffersystem eine Kombination aus einer schwachen Säure und der korrespondierenden Base darstellt. Als Kreatin-Komponente wird vorzugsweise Kreatin, Kreatin-Monohydrat und/oder mindestens ein Salz sowie eine Anlagerungs- oder Komplexverbindung davon eingesetzt. Besonders bevorzugt ist im Rahmen der vorliegenden Erfindung das mindestens eine Salz, die mindestens eine Anlagerungs- und/oder Komplexverbindung ausgewählt aus der Gruppe bestehend aus Apfelsäure, Ascorbinsäure, Bernsteinsäure, Brenztraubensäure, Fumarsäure, Asparginsäure, Gluconsäure, α-Ketoglutarsäure, Oxalsäure, Pyroglutaminsäure, 3-Nicotinsäure, Maleinsäure, Schwefelsäure, Essigsäure, Ameisensäure, Phosphorsäure, Salzsäure, 2-Hydroxybenzoesäure, α-Liponsäure, L-Carnitin, Acetyl-L-Carnitin, Taurin, Betain, Cholin und Methionin. In einer bevorzugten Ausführungsform liegt die Kreatin-Komponente fest, besonders als Pulver oder in wässriger Lösung vor.

Es ist als erfindungswesentlich anzusehen, dass das Puffersystem einen pH-Wert von 8,0 bis 12,0 und vorzugsweise 10,0 bis 11,0 einstellt. Als bevorzugtes Puffersystem sieht die vorliegende Erfindung eine Mischung aus Natriumcarbonat und Natriumhydrogencarbonat vor. Das Verhältnis der beiden Komponenten kann in weiten Bereichen frei gewählt werden, wobei dieses vorzugsweise so gewählt wird, dass der pH-Wert der Formulierung sich auf 10,0 bis 11,0 eingestellt. Hierbei ist es von Vorteil, dass bei der richtigen Wahl des Mischungsverhältnisses die Einsatzmenge praktisch nicht eingeschränkt wird. So stellt sich bei der Verwendung einer 1 zu 1 Mischung zwangsläufig ein pH-Wert von 10,4 ein, wobei der dieser unabhängig von der eingesetzten Gesamtmenge an Puffer ist.

Somit lässt sich ein aus organoleptischer Sicht akzeptabler pH-Wert einstellen, und gleichzeitig wird das Kreatin in optimaler Weise vor dem Einfluss von Säure geschützt, wodurch eine Umwandlung in Kreatinin vermieden wird.

Als weitere Puffersysteme kommen auch Mischungen aus Natriumhydrogenphosphat und Natriumphosphat oder L-Lysin und L-Lysin-Natriumsalz oder L-Arginin und L-Arginin-Natriumsalz in Frage, wobei das verwendete Verhältnis wiederum so gewählt wird, dass sich der pH-Wert der Formulierung vorzugsweise auf 10,0 bis 11,0 einstellt.

Die Formulierung ist bzgl. der Puffer-Komponente nicht limitiert, wobei insbesondere die Menge der Puffer-Komponente, in denen sie in der Zubereitung vorliegen kann, keine Einschränkung darstellt. Aus ernährungsphysiologischen Gründen werden allerdings Mengen empfohlen, die zwischen 0,1 und 90,0 Gew.-%, bezogen auf das Gesamtgewicht der Zubereitung, liegen. Besonders bevorzugt sind Mengen zwischen 2,5 und 15,0 Gew.-% und insbesondere 5,0 bis 10,0 Gew.-%, bezogen auf das Gesamtgewicht der Zubereitung.

Überraschend hat sich bei der Verwendung der beschriebenen Puffersysteme herausgestellt, dass diese nicht nur zu einem geringeren Abbau des Kreatins im Magen führen, sondern dass das verabreichte Kreatin auch besser in die Zellen aufgenommen wird. So konnte in einem Experiment gezeigt werden, dass die erfindungsgemäßen Formulierungen zu einem deutlich höheren Anstieg der Kreatin-Konzentrationen im Muskel führen als dies bei der Verwendung von Kreatin Monohydrat oder den bekannten alkalischen Formulierungen nach dem Stand der Technik der Fall ist.

In diesem Zusammenhang konnte gezeigt werden, dass der Natrium-Gehalt der Formulierung einen entscheidenden Einfluss auf die Bioverfügbarkeit und die Aufnahme von Kreatin in die Zellen hat. Dies erscheint aufgrund der Abhängigkeit des Kreatin-Transporters von Natrium-Ionen plausibel. Die Verwendung einer Mischung aus Kreatin und Natriumsalzen zur Verbesserung der Aufnahme von Kreatin in den Muskel wurde bisher noch nicht beschrieben und bietet gegenüber der bisherigen Praxis der Verwendung von hohen Kohlenhydrat- oder Protein-Dosen deutliche Vorteile.

Die vorliegende Erfindung sieht somit neben dem Puffersystem auch optional die Einarbeitung von einem oder mehreren weiteren physiologisch akzeptablen Natriumsalzen oder einem Gemisch davon in die erfindungsgemäßen Zubereitungen vor. Hierfür kommen beispielsweise Natriumchlorid, Natriumsulfat, Natriumacetat, Natriumcitrat, Natriumgfuconat, Natriumascorbat, Natriumpantothenat und Natriumlactat oder Mischungen dieser Salze in Frage.

Der Anteil dieser Natriumsalze ist relativ unkritisch, doch hat es sich als besonders vorteilhaft erwiesen, diese weiteren Natriumsalze in einer Menge von 0,1 bis 75,0 Gew.-%, insbesondere 5,0 bis 55,0 Gew.-% und besonders bevorzugt 10,0 bis 20,0 Gew.-%, bezogen auf das Gesamtgewicht der Zubereitung, einzusetzen.

Die Verwendung der beschrieben Puffersysteme erscheint somit ideal, da zum einen die Stabilität des Kreatins gegenüber Säuren erhöht und somit der Abbau von Kreatin im Magen vermieden wird. Weiterhin verbessern die enthaltenen Natrium-Ionen die Aufnahme in die Zellen, wobei dieser Effekt auch über die Zugabe von weiteren Natriumsalzen noch verstärkt werden kann.

Gemäß einer bevorzugten Ausführungsform enthält die erfindungsgemäße Zubereitung noch weitere physiologisch aktive Verbindungen, wie z. B. Kohlenhydrate, Fette, Aminosäuren, Proteine, Vitamine, Mineralstoffe, Spurenelemente sowie Derivate und Mischungen davon. Außerdem kann der erfindungsgemäßen Zubereitung zur Verbesserung der Bioverfügbarkeit noch α-Liponsäure und/oder Guanidinoessigsäure zugesetzt werden. Für den Fall, dass die erfindungsgemäße Zubereitung als wässrige Lösung eingesetzt wird, wird der Feststoffgehalt vorzugsweise auf 0,01 bis 14,0 Gew.-% eingestellt.

Ein weiterer Gegenstand der vorliegenden Erfindung ist ein Verfahren zur Herstellung der erfindungsgemäßen Zubereitung, wobei die Kreatin-Komponente vorgelegt wird, ein Puffersystem, bevorzugt eine Mischung einer schwachen Säure und der korrespondierenden Base eingearbeitet wird und ggf. weitere Natriumsalze, physiologisch aktive Verbindungen und/oder α-Liponsäure und/oder Guanidinoessigsäure zugegeben werden. Bevorzugt wird die Kreatin-Komponente als Pulver oder wässrige Lösung vorgelegt. Weiterhin wird das Puffersystem bevorzugt homogen eingearbeitet.

Einen bevorzugten Aspekt der Erfindung betrifft eine pharmazeutische Zusammensetzung umfassend die erfindungsgemäße Zubereitung sowie gegebenenfalls einen oder mehrere pharmazeutisch annehmbare Träger und/oder Hilfsstoffe. Die vorliegende Erfindung beansprucht des Weiteren die Verwendung der erfindungsgemäßen Zubereitung als Nahrungsergänzungsmittel. Insbesondere wird die Verwendung der beanspruchten Zubereitung als physiologisches Stärkungsmittel und in diesem Zusammenhang insbesondere in Form eines Funktionsnahrungsmittels (Functional Food) für Menschen berücksichtigt, wobei der Schul-, Sport-, Rekonvaleszenz- und/oder Geriatrie-Bereich im Vordergrund stehen.

Die beschrieben positiven Effekte entfalten die beschriebenen Formulierungen auch bei Tieren, so dass der Einsatz auch in diesem Bereich vorgesehen wird. Werden die beschriebenen Kreatin-Formulierungen als Futtermittelzusatz verwendet, ist insbesondere die Verabreichung an Zucht- und Masttiere sowie Tiere im Leistungssport als bevorzugt anzusehen und in diesem Zusammenhang besonders bevorzugt an Schweine, Pferde, Geflügel und Fische, wobei sich die Verwendung als Ersatzmittel für Tier- und/oder Fischmehl sowie daraus hergestellte Produkte als besonders geeignet erwiesen hat. Der Ersatz kann dabei als Teil- oder auch Vollersatz stattfinden.

Weiterhin kann die neue Kreatin-Zubereitung auch als Nahrungsergänzung oder Emährungsbestandteil für Haustiere wie Hunde, Katzen und Vögel eingesetzt werden.

Als Applikationsformen haben sich besonders Pulver, Granulate, Pastillen, Kapseln, Tabletten, Lösungen, Säfte und/oder Gelee-Produkte als besonders geeignet erwiesen. Dabei kann es in Abhängigkeit vom jeweiligen konkreten Verwendungsfall durchaus empfehlenswert sein, die erfindungsgemäße Zubereitung in Kombination mit anderen physiologisch aktiven Wirkstoffen einzusetzen.

Die erfindungsgemäße Zubereitung kann in Einzeldosen von 0,001 bis 0,3 g/kg Körpergewicht bzw. in Tagesdosen verabreicht werden, die zwischen 0,001 und 1,0 g/kg Körpergewicht liegen. Dies gilt insbesondere für die pharmazeutische Zusammensetzung sowie die Verwendung als Futtermittel, Nahrungsergänzungsmittel, physiologisches Stärkungsmittel, aber auch als Funktionsnahrungsmittel.

Insgesamt stellt die vorgeschlagene Formulierung und ihre Verwendung eine weitere Fortentwicklung des Standes der Technik hinsichtlich einer Erhöhung der Stabilität von Kreatin-Formulierungen dar. Als besonders vorteilhaft erwies sich darüber hinaus eine verbesserte Bioverfügbarkeit der Kreatin-Komponente.

Die nachfolgenden Beispiele veranschaulichen die Vorteile der vorliegenden Erfindung.

### Beispiele

### 1. Nahrungsergänzungsmittel

Nachfolgend sind typische Zusammensetzungen von neutral oder wohlschmeckenden Formulierungen aufgeführt, deren Bestandteile bei Raumtemperatur in 500 ml Fruchtsaft, Wasser, Joghurt und/oder Molke eingebracht werden.

| | | |
|---|---|---|
| 1.1 | 2.980 mg | Kreatin Monohydrat |
| | 150 mg | Natriumcarbonat |
| | 118 mg | Natriumhydrogencarbonat |
| | | |
| 1.2 | 1500 mg | Kreatin Monohydrat |
| | 400 mg | Natriumcarbonat |
| | 600 mg | Natriumhydrogencarbonat |
| | 100 mg | Natriumcitrat |
| | 2000 mg | Natriumchlorid |
| | | |
| 1.3 | 1500 mg | Kreatin Monohydrat |
| | 4000 mg | Natriumcarbonat |
| | 6000 mg | Natriumhydrogencarbonat |
| | 500 mg | Guanidinoessigsäure |
| | 500 mg | Betain |
| | 300 mg | α-Liponsäure |
| | 400 mg | (MgCO₃)₄·Mg(OH)₂·5H₂O = ca. 100 Mg |
| | 500 mg | Vitamin C |
| | | |
| 1.4 | 1.500 mg | Kreatin Monohydrat |
| | 750 mg | L-Arginin |
| | 250 mg | L-Arginin-Natriumsalz |
| | 1.000 mg | Glucosamin |
| | 300 mg | Chondroitinsulfat |
| | 500 mg | Methionin |
| | 3.100 mg | Creatinol-sulfat |
| | | |
| 1.5 | 750 mg | Kreatin Monohydrat |
| | 750 mg | L-Lysin |
| | 750 mg | L-Lysin-Natriumsalz |
| | 1.000 mg | Natriumascorbat |

### 2. Futtermittel

2.1 Eine Formulierung bestehend aus 2.000 mg Kreatincitrat, 5.000 mg Inulin, 3000 mg Natriumchlorid, 600 mg Natriumcarbonat und 700 mg Natriumhydrogencarbonat wurde in eine typische Rezeptur für Futterpellets zur Futterergänzung von Pferden eingebracht.
2.2 Eine Formulierung bestehend aus 7.000 mg Kreatin Monohydrat, 750 mg Camitintartrat, 100 mg Succrosestearat, 160 mg Talkum, 1.090 mg Fructose, 2000 mg Natriumcarbonat und 4700 mg Natriumhydrogencarbonat wurde in die Basismasse für Hundebisquits eingebracht.
2.3 Als Masterbatch wurde in eine handelsübliche Katzendosenfuttermischung homogen folgende Formulierung eingebracht: 3.000 mg Creatinol-Suffat, 3.000 mg Kreatin Monohydrat, 40 mg Magnesiumstearat, 25 mg Carboxymethylcellulose und 135 mg Lactose, 500 mg Natriumphosphat und 1500 mg Natriumhydrogenphosphat.

### 3. Verhalten gegenüber Säuren

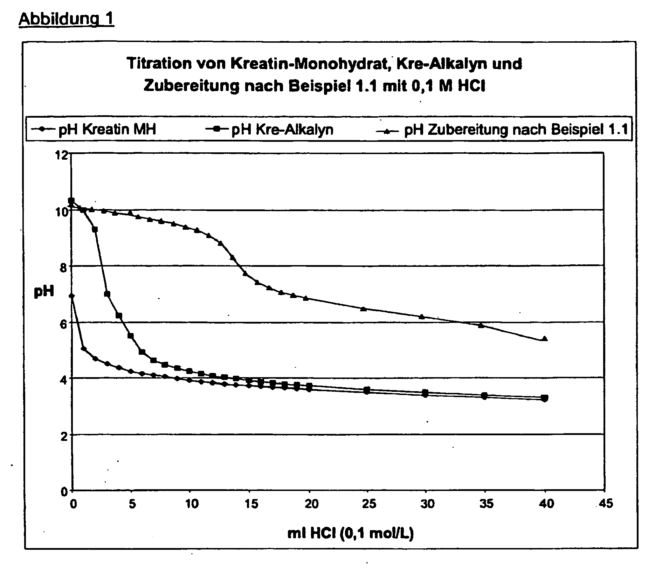

Der Einfluss der Zugabe einer starken Säure auf den pH-Wert einer Lösung einer erfindungsgemäßen Kreatin-Zubereitungen wurde untersucht und mit dem bisher auf dem Markt befindlichen alkalischen Kreatin-Zubereitungen (Kre-Alkalyn^{®}) und Kreatin-Monohydrat verglichen:
Kreatin Monohydrat und Kre-Alkalyn^{®} und eine erfindungsgemäße Kreatin-Zubereitung nach Beispiel 1.1 wurden in jeweils 500 ml Wasser gelöst. Die Menge wurde immer so gewählt, dass in jede Lösung 2980 mg Kreatin Monohydrat eingebracht wurde. Anschließend wurde mit 0,1 molarer Salzsäure titriert, wobei der pH-Verlauf mit einer pH-Elektrode gemessen wurde. Die erfindungsgemäße Formulierung tolerierte hierbei einen deutlich größere Zugabe von Säure, bevor diese in den sauren Bereich umschlug, wie aus Abbildung 1 deutlich zu entnehmen ist.

### Bioverfügbarkeit

Drei Probanden-Gruppen von jeweils zehn Personen wurden so zusammengestellt, dass in allen Gruppen etwa die gleichen durchschnittlichen Ausgangswerte von Kreatin in der Muskeltrockenmasse vorhanden waren.

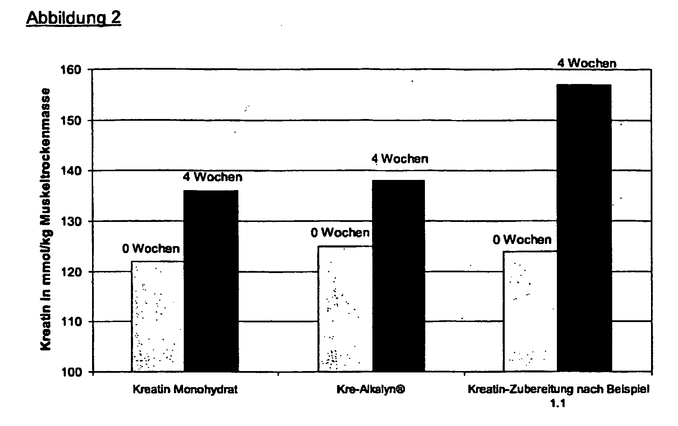

Über vier Wochen wurde den drei Gruppen täglich eine erfindungsgemäße Zubereitung nach Beispiel 1.1, Kreatin Monohydrat oder Kre-Alkalyn^{®} verabreicht. Die Dosis wurde dabei so gewählt, dass von jedem Proband pro Tag jeweils 2,0 g reines Kreatin Monohydrat aufgenommen wurde. Unmittelbar vor der Studie und zwei Wochen nach der Einnahme wurde der Kreatin-Gehalt im Muskel mittels Muskelbiopsie gemessen. Die Ergebnisse sind in Abbildung 2 dargestellt.

## Patentansprüche

1. Zubereitung, umfassend eine Kreatin-Komponente, **dadurch gekennzeichnet, dass** die Zubereitung zusätzlich ein Puffersystem bestehend aus einer Kombination aus schwacher Säure und korrespondierender Base, ausgewählt aus der Gruppe Natriumcarbonat/Natriumhydrogencarbonat, Natriumphosphat/Natriumhydrogen-phosphat, L-Lysin/L-Lysin-Natriumsalz und L-Arginin/L-Arginin-Natriumsalz, enthält, das einen pH-Wert von 8,0 bis 12,0 einstellt.

2. Zubereitung nach Anspruch 1, **dadurch gekennzeichnet, dass** die Kreatin-Komponente Kreatin, Kreatin-Monohydrat und/oder mindestens ein Salz, eine Anlagerungs- und/oder Komplexverbindung davon ist.

3. Zubereitung nach Anspruch 2, **dadurch gekennzeichnet, dass** das mindestens eine Salz, die mindestens eine Anlagerungs- und/oder Komplexverbindung ausgewählt ist aus der Gruppe bestehend aus Apfelsäure, Ascorbinsäure, Bernsteinsäure, Brenztraubensäure, Fumarsäure, Asparginsäure, Gluconsäure, α-Ketoglutarsäure, Oxalsäure, Pyroglutaminsäure, 3-Nicotinsäure, Maleinsäure, Schwefelsäure, Essigsäure, Ameisensäure, Phosphorsäure, Salzsäure, 2-Hydroxybenzoesäure, α-Liponsäure, L-Carnitin, Acetyl-L-Carnitin, Taurin, Betain, Cholin und Methionin.

4. Zubereitung nach einem der vorhergehenden Ansprüche, **dadurch gekennzeichnet, dass** der Anteil des Puffersystems zwischen 0,1 und 90,0 Gew.-%, bezogen auf das Gesamtgewicht der Zusammensetzung, liegt.

5. Zubereitung nach einem der vorhergehenden Ansprüche, **dadurch gekennzeichnet, dass** die Zubereitung zusätzlich ein Natriumsalz oder mehrere Natriumsalze oder ein Gemisch davon enthält.

6. Zubereitung nach Anspruch 5, **dadurch gekennzeichnet, dass** das eine Natriumsalz oder die mehreren Natriumsalze oder ein Gemisch davon ausgewählt ist aus der Gruppe bestehend aus Natriumchlorid, Natriumsulfat, Natriumacetat, Natriumcitrat, Natriumgluconat, Natriumascorbat, Natriumpantothenat und Natriumlactat.

7. Zubereitung nach Anspruch 5 oder 6, **dadurch gekennzeichnet, dass** der Anteil an einem Natriumsalz oder mehreren Natriumsalzen oder einem Gemisch davon 0,1 bis 75,0 Gew.-%, bezogen auf das Gesamtgewicht der Zubereitung, beträgt.

8. Zubereitung nach einem der vorhergehenden Ansprüche, **dadurch gekennzeichnet, dass** sie zusätzliche physiologisch aktive Verbindungen ausgewählt aus der Gruppe bestehend aus Kohlenhydraten, Fetten, Aminosäuren, Proteinen, Vitaminen, Mineralstoffen, Spurenelementen sowie Derivaten und Mischungen davon enthält.

9. Zubereitung nach einem der vorhergehenden Ansprüche, **dadurch gekennzeichnet, dass** sie zusätzlich Guanidinoessigsäure und/oder α-Liponsäure enthält.

10. Zubereitung nach einem der vorhergehenden Ansprüche, **dadurch gekennzeichnet, dass** das Puffersystem einen pH-Wert von 10,0 bis 11,0 einstellt.

11. Zubereitung nach einem der vorhergehenden Ansprüche, **dadurch gekennzeichnet, dass** sie fest oder wässrig ist.

12. Zubereitung nach einem der vorhergehenden Ansprüche, **dadurch gekennzeichnet, dass** die wässrige Zubereitung einen Feststoff-Gehalt von 0,01 bis 14,0 Gew.-%, bezogen auf das Gesamtgewicht der Zusammensetzung, aufweist.

13. Zubereitung nach einem der vorhergehenden Ansprüche, **dadurch gekennzeichnet, dass** sie als Pulver, Granulat, Pastille, Kapsel, Tablette, Lösung, Saft und/oder Gelee-Produkt vorliegt.

14. Verfahren zur Herstellung der Zubereitung nach einem der vorhergehenden Ansprüche, umfassend die Schritte, (a) Bereitstellen einer Kreatin-Komponente gemäß einem der Ansprüche 1 bis 3 definiert ist, (b) Einarbeiten eines Puffersystems, das gemäß einem der Ansprüche 1, 4 oder 10 definiert ist und (c) ggf. Hinzufügen von einem Natriumsalz oder mehreren Natriumsalzen oder einem Gemisch davon, das/die gemäß einem der Ansprüche 5 bis 7 definiert ist/sind, von physiologisch aktiven Verbindungen, die gemäß Anspruch 8 definiert sind, und/oder von Guanidinoessigsäure und/oder α-Liponsäure, die gemäß Anspruch 9 definiert sind.

15. Pharmazeutische Zusammensetzung umfassend eine Zubereitung nach einem der Ansprüche 1 bis 13, sowie ggf. mindestens einem pharmazeutisch annehmbaren Träger und/oder Hilfsstoff.

16. Nichttherapeutische Verwendung der Zubereitung nach einem der Ansprüche 1 bis 13 als Nahrungsergänzungsmittel, Futtermittel und/oder Futtermittelzusatz.

## Claims

1. A preparation comprising a creatine component, **characterized in that** the preparation additionally contains a buffer system comprising a combination of a weak acid and conjugate base, selected from the group of sodium carbonate/sodium hydrogen carbonate, sodium phosphate/sodium hydrogen phosphate, L-lysine/L-lysine sodium salt and L-arginine/L-arginine sodium salt which sets a pH of 8.0 to 12.0.

2. The preparation as claimed in claim 1, **characterized in that** the creatine component is creatine, creatine monohydrate and/or at least one salt, an addition compound and/or complex compound thereof.

3. The preparation as claimed in claim 2, **characterized in that** the at least one salt, the at least one addition compound and/or complex compound is selected from the group consisting of malic acid, ascorbic acid, succinic acid, pyruvic acid, fumaric acid, aspartic acid, gluconic acid, α-ketoglutaric acid, oxalic acid, pyroglutamic acid, 3-nicotinic acid, maleic acid, sulfuric acid, acetic acid, formic acid, phosphoric acid, hydrochloric acid, 2-hydroxybenzoic acid, α-lipoic acid, L-carnitine, acetyl-L-carnitine, taurine, betaine, choline and methionine.

4. The preparation as claimed in any of the preceding claims, **characterized in that** the fraction of the buffer system is between 0.1 and 90.0% by weight, based on the total weight of the composition.

5. The preparation as claimed in any of the preceding claims, **characterized in that** the preparation additionally contains a sodium salt or a plurality of sodium salts or a mixture thereof.

6. The preparation as claimed in claim 5, **characterized in that** the one sodium salt or the plurality of sodium salts or a mixture thereof is selected from the group consisting of sodium chloride, sodium sulfate, sodium acetate, sodium citrate, sodium gluconate, sodium ascorbate, sodium pantothenate and sodium lactate.

7. The preparation as claimed in claim 5 or 6, **characterized in that** the fraction of one sodium salt or a plurality of sodium salts or a mixture thereof is 0.1 to 75.0% by weight, based on the total weight of the preparation.

8. The preparation as claimed in any of the preceding claims, **characterized in that** it contains additional physiologically active compounds selected from the group consisting of carbohydrates, fats, amino acids, proteins, vitamins, minerals, trace elements and also derivatives and mixtures thereof.

9. The preparation as claimed in any of the preceding claims, **characterized in that** it additionally contains guanidinoacetic acid and/or α-lipoic acid.

10. The preparation as claimed in any of the preceding claims, **characterized in that** the buffer system sets a pH of 10.0 to 11.0.

11. The preparation as claimed in any of the preceding claims, **characterized in that** it is solid or aqueous.

12. The preparation as claimed in any of the preceding claims, **characterized in that** the aqueous preparation has a solids content of 0.01 to 14.0% by weight, based on the total weight of the composition.

13. The preparation as claimed in any of the preceding claims, **characterized in that** it is present as powders, granules, pastils, capsules, tablets, solutions, juices and/or jelly products.

14. A process for producing the preparation as claimed in any of the preceding claims comprising the steps (a) providing a creatine component defined according to any of claims 1 to 3, (b) incorporating a buffer system which is defined according to any of claims 1, 4 or 10, and (c) if appropriate addition of a sodium salt or a plurality of sodium salts or a mixture thereof which is/are defined according to any of claims 5 to 7 of physiologically active compounds which are defined according to claim 8 and/or of guanidinoacetic acid and/or α-lipoic acid which are defined according to claim 9.

15. A pharmaceutical composition comprising a preparation as claimed in any of claims 1 to 13, and also, if appropriate, at least one pharmaceutically acceptable carrier and/or auxiliary.

16. The non-therapeutic use of the preparation as claimed in any of claims 1 to 13 as dietary supplement, feedstuff and/or feedstuff additive.

## Revendications

1. Préparation comprenant un composant créatine, **caractérisée en ce que** la préparation contient en outre un système tampon consistant en une combinaison d'acide faible et de base correspondante, choisi dans le groupe carbonate de sodium/hydrogénocarbonate de sodium, phosphate de sodium/hydrogénophosphate de sodium, L-lysine/sel de sodium de L-lysine et L-arginine/sel de sodium de L-arginine, qui établit un pH de 8,0 à 12,0.

2. Préparation selon la revendication 1 **caractérisée en ce que** le composant créatine est la créatine, la créatine monohydratée et/ou au moins un sel, un composé d'addition et/ou complexe de celle-ci.

3. Préparation selon la revendication 2 **caractérisée en ce que** le au moins un sel, le au moins un composé d'addition et/ou complexe sont choisis dans le groupe consistant en l'acide malique, l'acide ascorbique, l'acide succinique, l'acide pyruvique, l'acide fumarique, l'acide aspartique, l'acide gluconique, l'acide α-cétoglutarique, l'acide oxalique, l'acide pyroglutamique, l'acide 3-nicotinique, l'acide maléique, l'acide sulfurique, l'acide acétique, l'acide formique, l'acide phosphorique, l'acide chlorhydrique, l'acide 2-hydroxybenzoïque, l'acide α-lipoïque, la L-carnitine, l'acétyl-L-carnitine, la taurine, la bétaïne, la choline et la méthionine.

4. Préparation selon l'une des revendications précédentes **caractérisée en ce que** la fraction du système tampon est située entre 0,1 et 90,0 % en poids, par rapport au poids total de la composition.

5. Préparation selon l'une des revendications précédentes **caractérisée en ce que** la préparation contient en outre un sel de sodium ou plusieurs sels de sodium ou un mélange de ceux-ci.

6. Préparation selon la revendication 5 **caractérisée en ce que** le un sel de sodium ou les plusieurs sels de sodium ou un mélange de ceux-ci sont choisis dans le groupe consistant en le chlorure de sodium, le sulfate de sodium, l'acétate de sodium, le citrate de sodium, le gluconate de sodium, l'ascorbate de sodium, le pantothénate de sodium et le lactate de sodium.

7. Préparation selon la revendication 5 ou 6 **caractérisée en ce que** la fraction d'un sel de sodium ou de plusieurs sels de sodium ou d'un mélange de ceux-ci est 0,1 à 75,0 % en poids par rapport au poids total de la préparation.

8. Préparation selon l'une des revendications précédentes **caractérisée en ce qu'**elle contient des composés physiologiquement actifs supplémentaires choisis dans le groupe consistant en les glucides, les graisses, les aminoacides, les protéines, les vitamines, les substances minérales, les éléments à l'état de traces ainsi que les dérivés et les mélanges de ceux-ci.

9. Préparation selon l'une des revendications précédentes **caractérisée en ce qu'**elle contient en outre de l'acide guanidinoacétique et/ou de l'acide α-lipoïque.

10. Préparation selon l'une des revendications précédentes **caractérisée en ce que** le système tampon établit un pH de 10,0 à 11,0.

11. Préparation selon l'une des revendications précédentes **caractérisée en ce qu'**elle est solide ou aqueuse.

12. Préparation selon l'une des revendications précédentes **caractérisée en ce que** la préparation aqueuse présente une teneur en solides de 0,01 à 14,0 % en poids par rapport au poids total de la composition.

13. Préparation selon l'une des revendications précédentes **caractérisée en ce qu'**elle est sous forme de poudre, de granulé, de pastille, de capsule, de comprimé, de solution, de sirop et/ou de produit de type gelée.

14. Procédé pour produire la préparation selon l'une des revendications précédentes comprenant les étapes (a) fourniture d'un composant créatine défini selon l'une des revendications 1 à 3, (b) incorporation d'un système tampon qui est défini selon l'une des revendications 1, 4 ou 10 et (c) éventuellement addition d'un sel de sodium ou de plusieurs sels de sodium ou d'un mélange de ceux-ci, qui est/sont définis selon l'une des revendications 5 à 7, de composés physiologiquement actifs, qui sont définis selon la revendication 8, et/ou d'acide guanidinoacétique et/ou d'acide α-lipoïque, qui sont définis selon la revendication 9.

15. Composition pharmaceutique comprenant une préparation selon l'une des revendications 1 à 13 ainsi éventuellement qu'au moins un vecteur et/ou adjuvant pharmaceutiquement acceptable.

16. Utilisation non thérapeutique de la préparation selon l'une des revendications 1 à 13 comme complément alimentaire, aliment pour animaux et/ou additif d'aliments pour animaux.
